# EUROPEAN PATENT SPECIFICATION

(11) **EP 1 487 267 B1**
(45) Date of publication and mention of the grant of the patent: **29.10.2008**
(21) Application number: 02721159.8
(22) Date of filing: 27.02.2002
(51) Int. Cl.: A61K 9/72, A01N 25/18, A61L 9/04

(54) **PATCH FOR THE RELEASE OF VOLATILE SUBSTANCES TO ENVIRONMENT**
PFLASTER ZUR FREISETZUNG VON FLÜCHTIGEN SUBSTANZEN IN DIE UMGEBUNG
PATCH POUR LA LIBERATION DE SUBSTANCES VOLATILES DANS UN MILIEU AMBIENT

(30) Priority: 28.02.2001 US 272178 P
(43) Date of publication of application: 22.12.2004
(73) Proprietor: LAVIPHARM S.A., 190 02 Peania Attica (GR)
(72) Inventor: FOTINOS, Spiros, Athens (GR)
(74) Representative: Froud, Clive
(86) International application number: PCT/US2002/005768
(87) International publication number: WO 2002/067677

(56) References cited:
- EP-A- 0 761 240
- WO-A-94/10842
- WO-A-99/65430
- GB-A- 2 260 494
- US-A- 5 071 704
- US-A- 5 656 282
- US-A- 5 928 661

## Description

### Technical Field and Background Art

The present invention relates to compositions, devices and methods for the release of volatile substances in the environment and applications thereof.

It is known in the prior art to treat a subject with an active substance by administering the agent using any of a number of different routes of administration. In humans, for example, delivery of active substances can performed by oral administration by means of tablets, liquids, by inhalation (sprays, powders, solutions etc.) or by injection.

Volatile substances with beneficial properties to humans, such as antiseptic, soothing, skin revitalization, relief of sinus congestion, insect repellant and general tonic qualities, have had a history of popular use. At present, volatile substances are generally delivered in the form of an oil (bath or massage), candle wax or spray using a vaporizer or a diffuser. These methods are time consuming or cumbersome, show a decline in compliance of the subject to follow a certain mode of application or require a non-portable equipment for delivery.

Active agents have also been delivered through the skin surface using a multilayer system (patch) that has an adhesive surface associated with a drug containing polymeric matrix, and a backing support. Patches have been designed for transdermal or topical delivery of therapeutic agents including those having volatile active ingredients such as tolbyterol, propanolol, verapamil deprenyl (US 5,462,746), agents for aromatherapy (WO 00/16752) and agents for treating nicotine addiction (WO 0033812, US 5,902,601). Other patches for delivery of volatile therapeutic agents are described in US 4,675,009, 5,536,263, 5,741,510, 6,096,333; and 6,096,334. The patches described in these references include flexible, hydrophilic pressure sensitive adhesive gelatinous reservoirs that include a hydrophilic hydrocolloid gel for sustained release of medication to be absorbed topically through the skin of a patient. The preferred design for volatile pharmaceutically active ingredients is to minimize loss of the active agent during preparation and to minimize loss of the agent into the environment away from the skin after application.

The above described devices and patches characteristically include an impermeable backing film on one face and on the opposite face an adhesive layer protected with a second impermeable release layer which is peelable. Before application to the skin, the release layer is removed exposing an adhesive layer for adhering to the skin of the subject. In between the backing layer and the adhesive layer there is commonly a matrix or reservoir layer containing the active agent.

Additionally, patches have been described for placing on a subject and releasing agents into the environment to act as deterrents for sharks (US 5,696,333) and as insect repellants. (US 5,656,282). The shark repellant releasing device releases the active agent at an appropriate time prior to attack by a shark when the shark is already nibbling on the diver's suit. In this case the repellant should not otherwise be dispersed in the water. Accordingly, the diver causes a removal of an impermeable membrane from the reservoir surface to liberate the contents of the reservoir.

An insect repellant containing device uses differential adhesion to separate a layer containing an absorbent substrate saturated with insect repellant fluid from a pouch on one surface while maintaining contact with an adhesive backing layer on the opposite surface. A device or patch, containing insect repellant would be useful for providing high concentrations of the volatile agent into the environment without producing an adverse reaction on the skin of the subject. For example, in excess of 35% of N-diethyl-meta-toluamide can cause rashes. Heavy use of concentrations in excess of 80% have been linked to short term schizophrenia, while behavior will return to normal after use is discontinued. Many people have noticed becoming irritable after extended use of high concentrations of DEET based repellants.

Reference may also be made to the following: US-A-5,071,704, which relates to a device for controlled release of vapors and scents: WO-A-94/10842, which relates to a device for defence against insects; WO-A-99/65430, which relates to adhesively applied external nasal strips and dilators containing medications and fragrances; GB-A-2 260 494, which relates to a gel type fragrant composition; US-A-5,928,661, which relates to a controlled release composition containing volatile compound; EP-A-0 761 240, which relates to a transparent gelatin gel type air freshener; and US-A-5,656,282, which relates to a package for containing and applying a bug repellant patch.

It would be desirable to provide a patch for releasing volatile substances into the environment which would be easy to use, and protective of the user from skin contact with the volatile substance and further to offer flexibility to manipulate release of the volatile agent over preselected periods of time.

### Summary of the invention

In a first embodiment of the invention there is provided a patch, that includes an effective amount of a volatile agent within a solid layer, the sold layer positioned between a breathable layer and a barrier layer, wherein the barrier layer is in contact with a first face of an adhesive layer, the adhesive layer contacting a second face, a release liner or wherein the barrier layer has adhesive properties so as to directly contact the release liner, the removal of the release liner exposing the adhesive barrier layer for adhesive contact with a surface of a subject or an inanimate object.

In further embodiments, the solid layer of the patch is non-hydrophilic. For example, such a solid layer may be formed with the volatile agent dispersed in a gelatin mixture, ozokerities wax or sodium stearate. Alternatively, dispersion of the volatile agent within the solid layer may be accomplished by microcapsules encapsulating the volatile agent or by microbeads covered with the volatile agent.

In further embodiments, the volatile agent may be released from the patch over a 24 hour period, a 12 hour period, a 6 hour period or a one hour period.

In further embodiments, the volatile agent may be an aromatherapy oil, an insect repellant, a deodorant, a perfume, or a therapeutic activity. Moreover, the therapeutic activity may be selected from reducing sinus congestion, having a sedative activity and having a mood altering activity.

In an embodiment of the invention, a method is provided for making a patch for release of volatile substances, that includes (a) mixing a volatile substance with a liquid agent wherein the agent forms a solid below 40°C and is capable of forming a liquid above 45°C and below 90°C; (b) applying the liquid mixture of step (a) onto a breathable layer and permitting the liquid to form a solid; (c) preparing a barrier layer for adhesive attachment to the breathable layer and solid; and laminating the barrier layer by adhesion to the solid and the breathable layer; and (d) laminating a release liner by adhesion to the barrier layer.

In further embodiments, the patch may be packaged within a sealed pouch for removal therefrom prior to application of the patch to a surface of a subject. The solid may include any of a gelatin or a wax. and the volatile substance may be selected from an aromatherapy oil, an insect repellant, a deodorant, a perfume or an agent with therapeutic activity. The therapeutic activity is selected from reducing sinus congestion, having a sedative activity and having a mood altering activity.

In a further embodiment of the invention, a method is provided for delivering a volatile substance to an environment from a surface location of a subject, that includes applying a patch adhesively to the surface location of the subject so as to deliver the volatile substance to the environment. The patch may include an effective amount of a volatile agent within a solid layer, the solid layer positioned between a breathable layer and a barrier layer, wherein the barrier layer is in contact with a first face of an adhesive layer, the adhesive layer contacting a second face, a release liner or wherein the barrier layer has adhesive properties so as to directly contact the release liner, the removal of the release liner exposing the adhesive barrier layer for adhesive contact with a surface of a subject or an inanimate object.

In a further embodiment of the invention, a method is provided for delivering a volatile substance to an environment from a surface location of a subject, that includes applying a patch adhesively to the surface location of the subject so as to deliver the volatile substance to the environment. The patch may include an effective amount of a volatile agent within a solid layer, the solid layer positioned between a breathable layer and a barrier layer, wherein the barrier layer is in contact with a first face of an adhesive layer, the adhesive layer contacting a second face, a release liner or wherein the barrier layer has adhesive properties so as to directly contact the release liner, the removal of the release liner exposing the adhesive barrier layer for adhesive contact with a surface of a subject or an inanimate object. Moreover, the patch may be made by a method that includes (a) mixing a volatile substance with a liquid agent wherein the agent forms a solid below 40°C and is capable of forming a liquid above 45°C and below 90°C; (b) applying the liquid mixture of step (a) onto a breathable layer and permitting the liquid to form a solid; (c) preparing a barrier layer for adhesive attachment to the breathable layer and solid; and laminating the barrier layer by adhesion to the solid and the breathable layer; and (d) laminating a release liner by adhesion to the barrier layer.

In further embodiments relating to the above described method of use of the patch the volatile substance may be selected from an aromatherapy oil, an insect repellant, a deodorant, a perfume or an agent with therapeutic activity and the therapeutic activity may be further selected from reducing sinus congestion, having a sedative activity and having a mood altering activity.

### Brief Description of the Drawings

The foregoing features of the invention will be more readily understood by reference to the following detailed description, taken with reference to the accompanying drawings, in which:

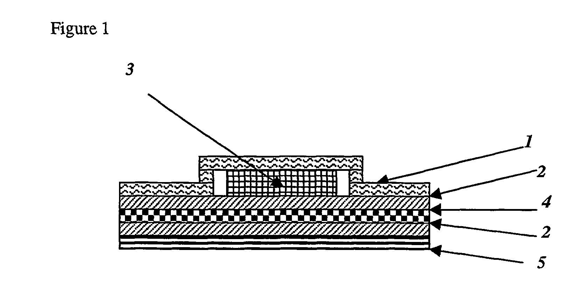

Figure 1 shows an embodiment of a patch of the present invention for release of volatile substances.

### Detailed Description of Specific Embodiments

Definitions. As used in this description and the accompanying claims, the following terms shall have the meanings indicated, unless the context otherwise requires:
"Patch" refers to any of a single layer film or film forming gel or multi-layered optionally laminated device for the delivery of agents including volatile substances included within the patch. The term "device" may include or alternatively consist essentially of a film. The term "device" may further include or alternatively consist essentially of a dosage unit.
" Volatile" refers to a boiling point that is less than water and a vapor pressure that is greater than water.
"Agent" or "substance" refers to a composition with a desirable or beneficial effect relating to a human subject.
"Natural" refers to anything derived from nature.
"Selected site" refers to any part of the body of a subject including a covering of a subject or a room space.
"Subject" refers to an animal, for example, a mammal, for example a human.
"Solid" refers to a state of matter which has a definite shape and offers resistance to a deforming force.

### The active agent

Embodiments of the invention refer to a patch for placing on a subject and releasing a volatile substance into the environment. The active agent may be a single natural or synthetic chemical compound or a mixture of compounds with volatile properties. Examples of volatile substances for incorporation in the patch includes agents for relief of sinus congestion, insect repellant, deodorizers and aromatherapy agents. Any aromatherapy agent known in the art may be used for the above uses in a patch as disclosed herein for example, Lavendar, Camomille, Clary Sage, Frankincense, Marjoram, Melissa, Neroli, Rose and Ylang, Ylang, Bergomot, Cajeput, Garlic, Manuka, Niaouli, Ravensara, Tea-tree, Geranium, Grapefruit, Jasmine, Mandarin, Mimosa, Orange, Petilgrain, Sandalwood, Myrrh, Juniper, Cardomon, Vetivert, Cedarwood, Cypress, Thyme, Peppermint, Rosemary, Eucalyptus, Litsea cubeba, Fennel, Birch, Hyssop, Benzoin, Lemon and Pimento and Ginger. Volatile compositions for use in a patch for acting as insect repellants include citronella, basil essential oil, geranium essential oil, lavender, rhodinol, geraniol, citronellol, citral, benzil, benzylbenzoate, N-butylacetanilide, Idalone, (FMC Corp, USA) N-diethyl-meta-toluamide (DEET), dimethyl carbate derivatives of cyclohexanol, 2-ethyl-2-butyl-1,3-propandiol, 2,5-dimethyl-2,5-hexandiol and similar higher alcohols, butyl esters or bicarboxylic acid of the formula H₉C₄-O-OC-(CH₂)n-CO-O-C₄H₉ where n is an integer between 2 and 6 and mixtures thereof and dimethyl pthalate or 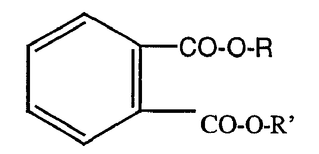 where R and R' are C₁-C₅ alkyles.

### Uses of the patch

The vapors of the volatile active agent may serve to bring about a feeling of well being, mood enhancement, sedation, and relaxation, including a feeling of relief from a condition such as sinus headache, small muscle tension, puffy edematous eyelids. The vapors of the volatile active agent may act so as to repel insects or parasites, to refresh the environment, to deter moths in stored clothes or to aromatize a room space and the like or to neutralize body odor.

In addition, the patch may include one or more of: an anti-irritant, a cleansing agent, a colorant, a filler, an antioxidant, a moisturizer, a skin conditioning agent, a solubilizer, a solvent, an anaesthetic agent, an anti-microbial, a cosmetic agent, a perfume and a therapeutic agent that is not a volatile substance and the like.

### Properties of the patch

One advantage of the patch described herein is that the active agent may be effective without contacting the skin of the subject. This avoids irritation and sensitization of the skin which occurs in at least a significant subset of the population of subjects when contacted with the active agent in liquid or solid form. Moreover, the patch provides a continuous and controlled release of the included volatile substance for desired time periods, for example, from as little as about one minute, or from about five minutes to one hour, or from one hour to four hours, or from four hours to twelve hours and from 12 hours to 24 hours and in another embodiment of the invention for at least 6 hours including embodiments desired to last for 12 hours or about 24 hours.

### Description of the patch

Referring to the drawing, Fig.1, one external face of the patch is a breathable layer 1 that may be a non-woven, a woven fabric or a plastic film that has been perforated. An example of a breathable layer includes non-woven fabric formed from a mixture of cellulose fibers derived from wood pulp and polyester fibers. The fibers are assembled loosely to form a layer that maintains porosity. A unifying or sizing resin is applied to hold the fibers together. One example is Hycar.RTM. 26477, an acrylate resin emulsion adhesive produced by B. F. Goodrich Co. of Brecksville, Ohio. The cellulose or polyester non-woven fabric may contain an acrylic latex emulsion resin as sizing, e.g., product number N7601 by Dexter Corporation of Windsor Locks, Conn. Non-woven breathable layers have some advantages relating for example to evaporation of volatile oils. A Bertek (breathable layers films). Moisture vapor transmission rate (MVTR). Other breathable layers may be used as described in the art-for example, EW-1900 (Estee Lauder), Freudenberg CO104, nylon point bound material (Cerex)

Adjacent to and extending into the non-woven layer is a solid layer 3 containing the volatile active substance. The volatile active substance is associated with a solid. The volatile active substance may be directly mixed within the solid or, in the alternative, provided in embedded microparticles. In this alternative, the volatile substance is contained within the core of the particles, dispersed throughout the particles or positioned on the surface of the particles. The solid 3 may be gelified (volatile substance incorporated in gelatin) or may contain wax (e.g., volatile substance incorporated in ozokerite wax).

Where the solid is a gelified layer, it may be formed from a long chain hydrocarbon or a polymer and may have properties of being a solid at room temperature but readily liquifying at temperatures below 100°C. The gelified layer may be formed from sodium stearate optionally containing glycerol, which is solid at room temperature but may be liquefied at 65-70°C for molding as desired. Where the solid is a wax such as for example ozokerities wax, the wax may have a low melting point and create a microcrystalline mixture.

Adjacent to the solid 3 layer and optionally sandwiched between two adhesive layers is a barrier layer 4, which cannot be penetrated by the volatile compound. The barrier layer 4 includes non-porous or non-occlusive materials which may be made of plastic, coated fabric, woven or non woven materials for example, paper, cellophane, polyethylene, polyester, polypropylene, polyurethane, polyvinyl chloride, polyamide or metallic foils such as aluminium foil. The barrier layer 4 may have but is not limited to a thickness of 1 to 5 mils (1/1000 of an inch is 1 mil).

The adhesive polymeric layer fixes the layer 3 with the volatile substance to the barrier layer 4. It may optionally be a separate layer 2 from the volatile agent containing layer or it may be the volatile agent containing layer. It can be made of synthetic adhesives such as acrylics, rubber, silicone, or other suitable materials that may have pressure sensitive properties. The adhesive layer 2 may be made from inert materials, which are further biologically and topically acceptable. Preferably, topically acceptable polymers with adhesion properties include acrylic based polymers such as GELVAO series sold by Monsanto, rubber based polymers such as DURO-TAKO series sold by National Starch and silicone-based polymers such as BIO-PSA X7-4302 SILICONE PSA sold by Dow Coming.

The adhesive polymeric layer may be formed from entirely natural materials for example, one or more volatile substance may be mixed with a polymer which is a plant protein such as prolamine (gliadin from wheat or zein from corn), and a plant polar lipid such as a ceramide, to form a dispersion. Other polysaccharides include as cellulose and cellulose derivatives; cyclodextran, gums such as arabic gum, tragacanth gum, chatti gum, karaya gum mastic gum; or gums produced by a microbial growth and fermentation such as xanthan gum, gellan gum. An entirely natural sheet, such as cellulose can be used. The volatile agent is dissolved in a suitable solvent such as a non-ionic water soluble polymer for example hydoxypropylcellulose by stirring at ambient temperature.

The adhesive polymer can consist of at least one layer of the adhesive -containing substances and/or additives. The adhesive polymer may be composed of more than one layer with a thickness in the range of 0.5-3.0 mils or more particularly 0.5-0.6 mils.

The adhesive layer 2 on the externals face of the barrier layer 4 may be protected by a release liner 5. The release liner 5 may be removed prior to application of the patch to expose the adhesive surface for adhering to the surface of the subject or onto an inanimate object. The release liner may be made from natural high impact polystyrene film (grade code 10106) sold by REXAM Release or a siliconized polyester film sold by REXAM Release. In accordance with a specific embodiment, the release liner is a "polyester" plastic coated with silicon anti-adherent coating (PET). The thickness of the release liner may be but is not limited to 3 to 10 mils.

In the embodiment provided in Figure 1, the barrier layer 4 is shown to be sandwiched between two adhesive layers 2 formed from Duro-Tak 87-6173, the first adhesive layer having a first face adjacent to and in contact with the breathable layer 1 and solid containing the volatile substance 3, and a second face adjacent to and in contact with the barrier layer 4, the second adhesive layer having a first face being adjacent to and in contact to the barrier layer 4 and a second face adjacent to and in contact with the release liner 5.

In another embodiment of the invention, a patch device can include a solid polymeric layer containing the volatile agent where the polymeric layer itself is adhesive. In this circumstance, the adhesive solid will be adjacent and in contact with the barrier layer absent an additional adhesive layer. If the barrier layer is adhesive then the barrier layer may be in direct contact with the release liner. Thus a patch may be formed that has more than four layers, where the 4 layered patch has a breathable layer, an adhesive solid layer containing volatile substance, a barrier layer and a release layer.

The size and the shape of the patch containing volatile agent may be designed to fit the site of application. The size, shape, and color of the device can be fanciful, or can be manufactured for minimal contrast with a shade of the skin. In an embodiment of the invention, the patch in the center portion has a thickness of approximately 33 or 34 mil and at the edges of the patch, the thickness is 21 or 22 mil. The thickness at the edges is kept thin by adhering the barrier layer to the breathable layer without a thick layer such as a sponge-like layer there between.

The patch may be packaged in a blister pack formed from material that is impermeable to gas or liquids and has an easy opening to provide access to the patch within. Optionally, the breathable layer which protects the gelified or other layer containing volatile agent and controlling release may be protected in the blister pack by a layer that is loosely adhered to the barrier to further protect the subject from contact with volatile substance and can be readily removed without separating the barrier layer from the patch.

The following examples are provided by way of illustrating embodiments of the invention but are not intended to be limiting.

### Examples

### Example 1: A patch containing a volatile substance in a solid layer

A patch containing a volatile substance in a solid layer was prepared as follows: A volatile substance was dissolved in a non-ionic water-soluble polymer, for example, hydroxypropylcellulose (KLUCEL by AQUALON Company Wilmington, DE. 19894 USA) by stirring at ambient temperature.

Using a coating device (square tool steel Multi Clearance Applicator sold by BYK Gardner) with a 0.5 mil casting gap, a layer of the gelified mixture was laminated on a non-woven backing substrate 1. This was identified as Roll A.

An adhesive polymeric layer 2 was laminated on one face onto a barrier layer 4 formed from polypropylene layer and dried in an oven at 60-80°C to form Roll B. The opposite side of the adhesive polymeric layer was laminated on a release liner 5.

The system thus obtained was delaminated by removing the release liner and further laminated with Roll A to result in the structures shown in Figure 1. The patch had the following dimensions: PET (polyester release layer) of 3 mil, adhesive DuroTak 87-6173 of 3 mil, polypropylene of 2mil, adhesive Durotak 87-6173 of 5mil, non-woven layer of 9mil, non-woven and solid wax layer was 9+3mil=12 mil.

### Example 2: Insect repellant patch

A patch was formed that included mosquito repellant as the volatile substance in a gelatin (180 bloom) layer. The preparation of a patch containing a volatile substance in gelatin is as follows: The gelatin was dissolved in hot de-ionized water at approx. 60°C under stirring. At this temperature, the mixture was liquid. Glycerine was added at 40°C. While cooling, the thickness of the mixture increased. The mosquito repellant was then added. The relative amounts of each component in the composition is shown below.

| ***Name of component*** | ***(%)*** |
|---|---|
| Gelatin (180 bloom) | 5 |
| Glycerin | 65 |
| H₂O | 10 |
| REPEL | 20 |

Using a coating device (square tool steel Multi Clearance Applicator sold by BYK Gardner) with a 0.5 mil casting gap, a layer of the mosquito repellant containing gelatin mixture was coated on a non-woven backing substrate (Fyber Dynamics, Inc.) to form Roll A1 as described in Example 1. Strips of about 2cm width were cut out.

An adhesive polymeric layer (Duro-Tak^{®} 87-6173, National Starch) was coated onto a release liner usually a polyester film, and was dried in an oven at 60-80°C and laminated with a barrier layer, for example, polypropylene layer.

Another release liner (polyester film again) was coated with an adhesive polymeric layer (Duro-Tak® 87-6173, National Starch), dried in the oven at 60-80°C for 30-60 min and laminated with the above roll (with the exposed side of the barrier film).

The system thus obtained is delaminated by removing the release liner and further laminated with the strips cut out from the Roll A1. On the top a layer of non-woven material was laminated on the adhesive layer (covering the non-woven which contains mosquito repellant) to result in the structure shown in Figure 1.

A second patch was formed as described above as a control patch where the gelatin layer did not include insect repellant (Placebo patch (PL-10401-A).

The results are shown in Table 1. With the Placebo patch (PL-10401-A), nine (9) out of 14 female mosquitos bit and had sucked blood, corresponding to 65% of female mosquitos. In contrast, using a commercially made patch having a different design from that disclosed herein (CER' 8^{®)}, 2 out of 20 female mosquitos that bit and had sucked blood, corresponding to 10% of female mosquitos. (CER' 8^{®} is produced by Larus Pharma, Italy which is a "natural emanating patch" consisting of 60mg of microcapsules with Citronella essential oil. (EP0669802, WO 94/10842). Using patches formed according to Example 1 and containing volatile substances of specific essential oils REP-10302, none of the 20 female mosquitos bit. Furthermore, the mosquitos were disabled.

When CIT-10301-A (citronella patch) made according to Example 1, two (2) out of 16 female mosquitos bit and had sucked blood, corresponding to 12.5% of female mosquitos.

### Example 3. Insect repellant patch

A patch containing a volatile substance in ozokerite (wax) was formed where the volatile substance was mosquito repellant. A patch was prepared that contained a volatile substance in an ozokerite (wax) as follows: The ozokerite (wax) was heated up to 55-65°C where it formed a liquid and mosquito repellant was added to the wax while stirring. While cooling, the mixture was coated onto a non-woven material. The procedure described in example 2 was then followed.

| **PATCH NAME** | **TEST TIME** | **NUMBER OF MOSQUITOS** | **NUMBER OF FEMALE MOSQUITOS** | **NUMBER OF STINGS** | **NUMBER OF FEMALE MOSQUITOS "SUCKED WITH BLOOD"** | **NUMBER OF FEMALE MOSQUITOS "NON-SUCKED WITH BLOOD"** |
|---|---|---|---|---|---|---|
| **PL-10401-A** | **11:40-12:00** | **24** | **5** | **2** | **2** | **3** |
| **(PLACEBO)** | | **24** | **9** | **7** | **7** | **2** |
| | | *Total: 48* | *Total: 14* | *Total: 9* | *Total: 9* | *Total: 5* |
| **REP-10302-A** | **14:55-15:15** | **40** | **8** | **0** | **0** | **8** |
| | **15:55-16:15** | **24** | **12** | **0** | **0** | **12** |
| | | *Total: 64* | *Total: 20* | *Total: 0* | *Total: 0* | *Total: 20* |
| **CIT-10301-A** | **16:55-17:15** | **20** | **6** | **1** | **1** | **5** |
| | **17:55-18:15** | **24** | **10** | **1** | **1** | **9** |
| | | *Total: 44* | *Total: 16* | *Total: 2* | *Total: 2* | *Total: 14* |
| **CER'8**® | **13:55-14:15** | **20** | **9** | **1** | **1** | **8** |
| | **16:35-16:55** | **18** | **11** | **1** | **1** | **10** |
| | | *Total: 38* | *Total: 20* | *Total: 2* | *Total: 2* | *Total: 20* |

## Claims

1. A patch comprising:
a breathable layer;
a barrier layer;
an effective amount of a volatile agent within a solid layer, the solid layer positioned between the breathable layer and the barrier layer, wherein the barrier layer is impermeable to the volatile agent;
an adhesive layer between adjacent sides of the solid layer and the barrier layer;
and
a release liner removably adhered to the barrier layer.

2. A patch according to claim 1 wherein the solid layer is non-hydrophilic.

3. A patch according to claim 1 wherein the solid layer comprises a gelatin mixture.

4. A patch according to claim 1 wherein the solid layer comprises ozokerities wax.

5. A patch according to claim 1 wherein the solid layer comprises sodium stearate.

6. A patch according to claim 1 wherein the volatile agent is released from the patch over a period of at least, six hours.

7. A patch according to claim 1 wherein the volatile agent is an aromatherapy oil.

8. A patch according to claim 1 wherein the volatile agent is an insect repellant.

9. A patch according to claim 1 wherein the Volatile agent is a deodorant.

10. A patch according to claim 1 wherein the volatile agent is a perfurme.

11. A patch according to claim 1 wherein the volatile agent has a therapeutic effect of reducing sinus congestion.

12. A patch according to claim 1 wherein the volatile agent has a sedative effect.

13. A patch according to claim 1 wherein the volatile agent has a mood altering effect.

14. A patch according to claim 1 wherein it is obtainable by a method according to claim 17.

15. A patch according to claim 1 wherein it further comprises a sealed pouch enclosing the patch prior to application of the patch to a surface of the subject.

16. A patch according to claim 1 wherein the breathable layer includes a non-woven fabric formed from a mixture of cellulose fibers from wood pulp and polyester fibers and resin.

17. A method fur making a patch for release of volatile substances comprising:
mixing a volatile substance with a liquid agent, wherein the agent forms a solid below 40°C and is capable of forming a liquid above 45°C and below 90°C;
applying the resulting liquid mixture onto a breathable layer and permitting the liquid to form a solid;
preparing a barrier layer for adhesive attachment to the breathable layer and solid;
laminating the barrier layer by adhesion to the solid and the breathable layer;
and
laminating a release liner by adhesion to the barrier layer.

18. A method according to claim 17 wherein it further comprises packaging the patch within a sealed pouch for removal therefrom prior to application of the patch to a surface of a subject.

19. A method according to claim 17 wherein the solid further comprises any of a gelatin, a wax or sodium stearate.

20. A method according to claim 17 wherein the volatile substance is selected from an aromatherapy oil, an insect repellent, a deodorant, a perfume or an agent with therapeutic activity.

21. A method according to claim 17 wherein the volatile substance has a therapeutic activity selected from reducing sinus congestion, having a sedative activity and having a mood altering activity.

22. A method or delivering a volatile non-therapeutic substance to an environment from a surface location of a subject, comprising applying a patch according to claim 1 adhesively to the surface location of the subject so as to deliver the volatile substance to the environment.

23. A method of delivering a volatile non-therapeutic substance to an environment from a surface location of a subject, comprising applying a patch made according to claim 17 adhesively to the surface location of the subject so as to deliver the volatile substance to the environment.

24. A method according to claim 22 or claim 23 wherein the volatile non-therapeutic substance is selected from an aromatherapy oil, an insect repellant, a deodorant or a perfume.

## Patentansprüche

1. Pflaster, umfassend:
eine atmungsfähige Schicht;
eine Sperrschicht;
eine wirksame Menge eines flüchtigen Mittels in einer Festschicht, wobei die Festschicht zwischen der atmungsfähigen Schicht und der Sperrschicht positioniert ist, wobei die Sperrschicht für das flüchtige Mittel undurchlässig ist;
eine Klebeschicht zwischen den angrenzenden Seiten der Festschicht und der Sperrschicht; und
ein Freisetzungsband, das an der Sperrschicht anhaftet.

2. Pflaster nach Anspruch 1, wobei die Festschicht nicht hydrophil ist.

3. Pflaster nach Anspruch 1, wobei die Festschicht eine Gelatinemischung aufweist.

4. Pflaster nach Anspruch 1, wobei die Festschicht Ozokeritwachs aufweist.

5. Pflaster nach Anspruch 1, wobei die Festschicht Natriumstearat aufweist.

6. Pflaster nach Anspruch 1, wobei das flüchtige Mittel vom Pflaster über einen Zeitraum von zumindest sechs Stunden freigesetzt wird.

7. Pflaster nach Anspruch 1, wobei das flüchtige Mittel ein Aromatherapieöl ist.

8. Pflaster nach Anspruch 1, wobei das flüssige Mittel ein Insektenrepellent ist.

9. Pflaster nach Anspruch 1, wobei das flüchtige Mittel ein Deodorant ist.

10. Pflaster nach Anspruch 1, wobei das flüchtige Mittel ein Parfüm ist.

11. Pflaster nach Anspruch 1, wobei das flüchtige Mittel eine therapeutische Wirkung bei der Verringerung der Sinus-Congestion besitzt.

12. Pflaster nach Anspruch 1, wobei das flüchtige Mittel eine sedative Wirkung besitzt.

13. Pflaster nach Anspruch 1, wobei das flüchtige Mittel eine die Stimmung ändernde Wirkung besitzt.

14. Pflaster nach Anspruch 1, wobei es durch ein Verfahren nach Anspruch 17 erhältlich ist.

15. Pflaster nach Anspruch 1, welches weiterhin einen verschlossenen Beutel umfasst, welcher das Pflaster vor Anwendung des Pflasters auf einer Oberfläche der Person enthält.

16. Pflaster nach Anspruch 1, wobei die atmungsfähige Schicht ein Fließgewebe enthält, gebildet aus einer Mischung an Zellulosefasern aus Holzbrei und Polyesterfasern und Harz.

17. Verfahren zur Herstellung eines Pflasters zur Freisetzung flüchtiger Substanzen, umfassend:
Vermischen einer flüchtigen Substanz mit einem flüssigen Mittel, wobei das Mittel einen Feststoff bei unter 40°C ausbildet und es zur Bildung einer Flüssigkeit bei über 45°C und unter 90°C befähigt ist;
Aufbringen der so erhaltenen flüssigen Mischung auf eine atmungsfähige Schicht, wobei der Flüssigkeit ermöglicht wird, einen Feststoff auszubilden;
Erstellen einer Sperrschicht zur klebenden Verbindung an die atmungsfähige Schicht und den Feststoff;
Laminieren der Sperrschicht durch Adhäsion an den Feststoff und die atmungsfähige Schicht;
und
Laminieren eines Freisetzungsbandes durch Adhäsion an die Sperrschicht.

18. Verfahren nach Anspruch 17, welches weiterhin das Verpacken des Pflasters in einen verschlossenen Beutel umfasst, aus dem das Pflaster vor Anwendung auf einer Oberfläche einer Person entfernt werden kann.

19. Verfahren nach Anspruch 17, wobei der Feststoff weiterhin eine Gelatine, ein Wachs oder ein Natriumstearat aufweist.

20. Verfahren nach Anspruch 17, wobei die flüchtige Substanz ausgewählt wird aus einem Aromatherapieöl, einem Insektenrepellent, einem Deodorant, einem Parfüm oder einem Mittel mit therapeutischer Wirkung.

21. Verfahren nach Anspruch 17, wobei die flüchtige Substanz eine therapeutische Aktivität besitzt, ausgewählt aus einer Verringerung der Sinus-Congestion, einem Mittel mit sedativer Wirkung und einem Mittel mit einer die Stimmung verändernden Wirkung.

22. Verfahren zur Bereitstellung einer flüchtigen nicht therapeutischen Substanz auf einer Umgebung einer Oberfläche einer Person, umfassend das Aufbringen eines Pflasters nach Anspruch 1 in lebender Form auf die Oberfläche der Person, um die flüchtige Substanz an die Umgebung bereitzustellen.

23. Verfahren zum Bereitstellen einer flüchtigen nicht therapeutischen Substanz an die Umgebung einer Oberfläche einer Person, umfassend das Aufbringen eines Pflasters nach Anspruch 17 in klebender Weise auf die Oberfläche der Person, um die flüchtige Substanz an die Umgebung bereitzustellen.

24. Verfahren nach Anspruch 22 und 23, wobei die flüchtige nicht therapeutische Substanz ausgewählt wird aus einem Aromatherapieöl, einem Insektenrepellent, einem Deodorant oder einem Parfüm.

## Revendications

1. Patch comprenant :
une couche perméable à l'air ;
une couche barrière ;
une quantité efficace d'un agent volatil dans une couche solide, la couche solide étant positionnée entre la couche perméable à l'air et la couche barrière, où la couche barrière est imperméable à l'agent volatil ;
une couche adhésive entre les côtés adjacents de la couche solide et la couche barrière ; et
un revêtement de libération adhérant de manière détachable à la couche barrière.

2. Patch selon la revendication 1, dans lequel la couche solide est non hydrophile.

3. Patch selon la revendication 1, dans lequel la couche solide comprend un mélange à base de gélatine.

4. Patch selon la revendication 1, dans lequel la couche solide comprend une cire ozocérite.

5. Patch selon la revendication 1, dans lequel la couche solide comprend du stéarate de sodium.

6. Patch selon la revendication 1, dans lequel l'agent volatil est libéré à partir du patch pendant une période d'au moins six heures.

7. Patch selon la revendication 1, dans lequel l'agent volatil est une huile aromatique.

8. Patch selon la revendication 1, dans lequel l'agent volatil est un insectifuge.

9. Patch selon la revendication 1, dans lequel l'agent volatil est un déodorant.

10. Patch selon la revendication 1, dans lequel l'agent volatil est un parfum.

11. Patch selon la revendication 1, dans lequel l'agent volatil a un effet thérapeutique de réduction de la congestion des sinus.

12. Patch selon la revendication 1, dans lequel l'agent volatil a un effet sédatif.

13. Patch selon la revendication 1, dans lequel l'agent volatil a un effet modifiant l'humeur.

14. Patch selon la revendication 1, dans lequel il peut être obtenu par un procédé selon la revendication 17.

15. Patch selon la revendication 1, dans lequel il comprend en outre un sachet hermétiquement fermé renfermant le patch avant l'application du patch sur une surface du sujet.

16. Patch selon la revendication 1, dans lequel la couche perméable à l'air comprend un tissu non tissé formé d'un mélange de fibres de cellulose de pâte de bois et de fibres polyester et d'une résine.

17. Procédé de préparation d'un patch pour la libération de substances volatiles comprenant :
le mélange d'une substance volatile avec un agent liquide, l'agent formant un solide au-dessous de 40 °C et étant capable de former un liquide au-dessus de 45 °C et au-dessous de 90 °C ;
l'application du mélange liquide résultant sur une couche perméable à l'air et le fait de laisser le liquide former un solide ;
la préparation d'une couche barrière pour un attachement adhésif à la couche perméable à l'air et au solide ;
la stratification de la couche barrière par adhérence au solide et à la couche perméable à l'air ; et
la stratification d'un revêtement de libération par adhérence à la couche barrière.

18. Procédé selon la revendication 17, dans lequel il comprend en outre l'emballage du patch dans un sachet hermétiquement fermé pour un retrait de celui-ci avant l'application du patch sur une surface d'un sujet.

19. Procédé selon la revendication 17, dans lequel le solide comprend en outre l'un quelconque d'une gélatine, d'une cire ou du stéarate de sodium.

20. Procédé selon la revendication 17, dans lequel la substance volatile est choisie parmi une huile aromatique, un insectifuge, un déodorant, un parfum ou un agent ayant une activité thérapeutique.

21. Procédé selon la revendication 17, dans lequel la substance volatile a une activité thérapeutique choisie parmi la réduction de la congestion des sinus, une activité sédative et une activité modifiant l'humeur.

22. Procédé de délivrance d'une substance volatile non thérapeutique à un environnement à partir d'une zone superficielle d'un sujet, comprenant l'application d'un patch selon la revendication 1 de manière adhésive à la zone superficielle du sujet de sorte à délivrer la substance volatile à l'environnement.

23. Procédé de délivrance d'une substance volatile non thérapeutique à un environnement à partir d'une zone superficielle d'un sujet, comprenant l'application d'un patch préparé selon la revendication 17 de manière adhésive à la zone superficielle du sujet de sorte à délivrer la substance volatile à l'environnement.

24. Procédé selon la revendication 22 ou la revendication 23, dans lequel la substance volatile non thérapeutique est choisie parmi une huile aromatique, un insectifuge, un déodorant ou un parfum.
